(19) Europäisches Patentamt
European Patent Office
Office européen des brevets

(11) Veröffentlichungsnummer: **0 291 751 B1**

(12) **EUROPÄISCHE PATENTSCHRIFT**

(45) Veröffentlichungstag der Patentschrift: **11.03.92**

(21) Anmeldenummer: **88106961.1**

(22) Anmeldetag: **30.04.88**

(51) Int. Cl.[5]: **A61K 33/06**, A61K 33/10, A61K 31/51, A61K 31/135, //(A61K33/10,33:06,31:51, 31:135)

# (54) Pharmazeutisches Kombinationspräparat.

(30) Priorität: **20.05.87 DE 3716863**

(43) Veröffentlichungstag der Anmeldung:
**23.11.88 Patentblatt 88/47**

(45) Bekanntmachung des Hinweises auf die Patenterteilung:
**11.03.92 Patentblatt 92/11**

(84) Benannte Vertragsstaaten:
**AT BE CH DE FR GB IT LI NL**

(56) Entgegenhaltungen:
**DE-A- 1 792 228**
**DE-A- 1 925 699**
**US-A- 3 279 997**

(73) Patentinhaber: **Wilhelm, Gerhard, Dr.**
**Stahlbühlring 80**
**W-6802 Ladenburg(DE)**

(72) Erfinder: **Wilhelm, Gerhard, Dr.**
**Stahlbühlring 80**
**W-6802 Ladenburg(DE)**

**EP 0 291 751 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein pharmazeutisches Kombinationspräparat zur Behandlung von allergischen Reaktionen, insbesondere von Heuschnupfen.

Heuschnupfen ist eine weitverbreitete allergische Reaktion des menschlichen Körpers, insbesondere der Schleimhäute im Nasen- und Augenbereich auf die Pollen bestimmter Pflanzen, besonders auf Pollen von Gräsern und Getreidearten. Die Allergie beeinträchtigt das Wohlbefinden und die Leistungsfähigkeit der Betroffenen sehr stark. Man versucht daher, mit verschiedenen Methoden, dieser Krankheit Herr zu werden, oder zumindest ihre Symptome so zu unterdrücken, daß auch der Aufenthalt der Patienten in der freien Natur ohne Beschwerden möglich ist.

Die außer der, wegen ihrer Nebenwirkungen weniger geschätzten Cortison-Behandlung, beiden verbreitetsten Behandlungsmethoden sind die sogenannte Desensibilisierung und die Verwendung von Antihistaminika.

Die Desensibilisierung bedingt einen hohen Aufwand, der zudem noch einen rechtzeitigen Behandlungsbeginn voraussetzt und der nicht immer erfolgreich ist. So ist es einleuchtend, daß wohl die meisten von Heuschnupfen befallenden Personen lieber während der Dauer der jährlich wiederkehrenden Heuschnupfenzeit Antihistaminika einnehmen.
Leider haben diese den großen Nachteil, daß sie den ohnehin schon angegriffenen Patienten müde machen. Ganz besonders schlimm ist dies, wenn die Betroffenen noch unter niedrigen Blutdruck leiden, was häufig der Fall ist.

Es bestand daher die Aufgabe, ein gut verträgliches Mittel zu finden, das im Bedarfsfalle einfach angewandt werden kann und das die Symptome des Heuschnupfens wirksam vermindert, ohne den Patienten zu belasten oder zu ermüden.
Die Lösung der Aufgabe erfolgt durch eine pharmazeutische Komposition gemäß der Ansprüche 1 bis 5.

Es ist aus DE-OS 17 92 228 und DE-OS 19 25 699 bekannt, daß Hydroxiphenylalkanolamine, gegen Heuschnupfen wirksam sind.

Leider ist die Wirkung dieser ansonsten gut verträglichen Stoffe nicht so gut wie sie von einem wirksamen Arzneimittel dieser Indikation verlangt wird. Eine Verbesserung der Wirksamkeit wird durch die aus DE-OS 31 29 872 bekannte Kombination der Hydroxiphenylalkanolamine mit einem Calciumsalz und Ascorbinsäure erzielt. Aber auch diese Wirksamkeit ist bei stark unter Heuschnupfen leidenden Patienten nicht ausreichend. Insbesondere wird auch durch eine Erhöhung der Einzeldosen eines derartigen Präparates die Wirkung nicht entsprechend erhöht.

Überraschenderweise wurde nun gefunden, daß ein Kombinationspräparat, das ein oder mehrere Hydroxiphenylalkanolamine der allgemeinen Formel

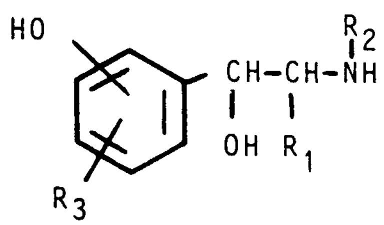

wobei die OH-Gruppe in m- oder p-Stellung steht und $R_1$ und $R_2$ gleich oder verschieden sind und Wasserstoff, eine Methyl-, Ethyl-, oder Isopropylgruppe und $R_3$ Wasserstoff, eine Hydroxyl-, Methyl- oder Methoxigruppe bedeuten, sowie Vitamin $B_1$ und ein Magnesiumsalz enthält, auch bei stark leidenden Patienten die Symptome des Heuschnupfens rasch und relativ langzeitig beseitigt.

Im allgemeinen reichen zwei Applikationen, um einen Patienten für 24 h beschwerdefrei zu halten. Werden die Hydroxiphenylalkanolamine in einer Retard- oder Depotform eingenommen, so reicht eine Applikation auch bei starker Polleneinwirkung für 24 h.
Die gegenüber der aus DE-OS 31 29 872 bekannten Kombination wesentlich verbesserte Wirkung der erfindungsgemäßen Komposition ergibt sich lediglich aus einer synergistischen Steigerung der drei Komponenten Hydroxiphenylalkanolamine, Vitamin $B_1$ und Magnesiumsalz.
Vitamin $B_1$ und Magnesiumsalz haben weder alleine noch gemeinsam miteinander eine die Symptome des Heuschnupfens lindernde Wirkung.

Eine wesentliche, weitere Steigerung ergibt sich durch die zusätzliche Kombination mit einem Magnesiumsalz. Zusätzliche Einnahmen weiterer biologisch aktiver Salze oder Vitamine wie etwa Calciumsalze, Vitamin C, $B_2$ oder $B_6$ bedingen keine feststellbare Änderung der erfindungsgemäßen Komposition hinsichtlich ihrer Wirkung gegen Heuschnupfen.
Hydroxiphenylalkanolamine bzw. deren wasserlösliche Salze sind als gut verträgliche, Herz und Kreislauf stabilisierende oral einzunehmende Mittel bekannt. Beispiele für diese Wirkstoffe sind
2-Ethylamino-1-(3-hydroxiphenyl)-ethanol
2-Amino-1-(3-hydroxi-phenyl)-propanol
2-Methylamino-1-(4-hydroxi-phenyl)-ethanol
2-Methylamino-1-(3-hydroxi-phenyl)-ethanol, oder
2-Amino-1-(3-hydroxi-phenyl)-ethanol.

Das erfindungsgemäße Kombinationspräparat enthält 2,5 bis 30 mg, bevorzugt 10 bis 25 mg eines oder mehrere der Hydroxiphenylalkanolamine, bevorzugt als wasserlösliches Salz. Dabei kann das Hydroxiphenylalkanolamin gelöst oder fest vorliegen. Weitere Vorteile hinsichtlich einer Langzeitwirkung des Präparates ergeben sich, wenn die Hydroxiphenylalkanolamin-Komponente in einer Retard-oder Depot-Form vorliegt. Dadurch, daß die Hydroxiphenylalkanolamine kreislaufstabilisierend und aktivitätssteigernd wirken, entfallen die bei den bisherigen kurzzeitig wirkenden Heuschnupfenmitteln beobachteten Ermüdungserscheinungen und darüber hinaus wird eine belebende Wirkung erreicht, die eine durch die Allergie bedingte Abgespanntheit ausgleicht, ohne daß irgendwelche Aufputschmittel verwendet werden müssen. Dies ist besonders wichtig, da sehr viele von Heuschnupfen geplagte Patienten unter Hypotonie oder hypotonen Kreislaufregulationsstörungen leiden. Weiterer Bestandteil des erfindungsgemäßen Kombinationspräparates ist Vitamin $B_1$, das in einer Menge von 20 bis 300 mg, bevorzugt von 50 bis 100 mg eingesetzt werden kann.

Der dritte notwendige Bestandteil ist ein wasserlösliches, nicht toxisches Magnesiumsalz, wie z. B. Magnesium-chlorid, -sulfat, -lactat, -gluconat, -ascorbat, -pantothenat oder -citrat, das in einer Menge von 100 bis 500 mg, bevorzugt von 150 bis 300 mg eingesetzt werden kann.

Die drei Komponenten der erfindungsgemäßen Komposition, die gegebenenfalls mit üblichen pharmazeutischen Trägern und Hilfsstoffen kombiniert werden können, liegen entweder im Wasser gelöst als verdünnte Lösung oder Sirup vor oder fest in Form einer Tablette, Dragée oder Kapsel. Letzteres empfiehlt sich insbesondere dann, wenn die Hydroxiphenylalkanolamin-Komponente oder das gesamte Präparat als Depot- oder Retard-Form vorliegt.

Beispiele:

Mehrere Patienten, die stark unter allergischen Reaktionen gegen Graspollen leiden, testeten die folgenden erfindungsgemäßen Kombinationen, sowie die Vergleichskombination jeweils an Tagen mit starkem Pollenflug.

Kombination I

25 mg 2-Ethylamino-1-(3-hydroxiphenyl)-ethanol.HCl
100 mg Vitamin $B_1$
200 mg Magnesiumcarbonat
gelöst in 100 ml Wasser

Kombination II

25 mg 2-Ethylamino-1-(3-hydroxiphenyl)-ethanol.HCl in handelsüblicher Depotform verkapselt
100 mg Vitamin $B_1$ als handelsübliches Dragée
1 handelsübliche Magnesium-Kautablette, enthaltend ein Magnesiumsalz entsprechend 115 mg $Mg^{++}$.

Diese drei Präparate werden zum gleichen Zeitpunkt eingenommen und stellen die erfindungsgemäße Kombination dar.

Kombination III

25 mg 2-Amino-1-(3-hydroxiphenyl)-ethanol.HCl
300 mg Magnesiumcitrat
150 mg Vitamin $B_1$

Vergleichskombination

75 mg 2-Ethylamino-1-(3-hydroxiphenyl)-ethanol.HCl
100 mg Ascorbinsäure
500 mg Calcium-gluconat
350 mg Calcium-lactat

| Ergebnis | | | | |
|---|---|---|---|---|
| | I | II | III | Vergleich |
| Beginn der Wirkung | 45 min | 60 min | 45 min | 45 min |
| Dauer der Wirkung | 14 h | 24 h | 14 h | 12 h |
| Beschwerdefreiheit im Stadtbereich | ja | ja | ja | ja |
| in ländlichen Bereichen | ja | ja | ja | nein |
| in Getreidefeldern | ja | ja | ja | nein |

## Patentansprüche

**1.** Pharmazeutisches Kombinationspräparat, **dadurch gekennzeichnet**, daß es eine Mischung aus einem oder mehreren Hydroxiphenylalkanolaminen der allgemeinen Formel

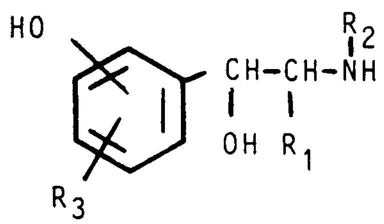

wobei die OH-Gruppe in m- oder p-Stellung steht und $R_1$ und $R_2$ gleich oder verschieden sind und Wasserstoff, eine Methyl-, Ethyl- oder Isopropylgruppe und $R_3$ Wasserstoff, eine Hydroxyl-, Methyl- oder Methoxigruppe bedeuten, sowie Vitaminen $B_1$ und ein Magnesiumsalz enthält.

**2.** Pharmazeutisches Kombinationspräparat nach Anspruch 1, **dadurch gekennzeichnet**, daß es die Hydroxiphenylalkanolamine als wasserlösliche Salze enthält.

**3.** Pharmazeutisches Kombinationspräparat nach den Ansprüchen 1 und 2, **dadurch gekennzeichnet**, daß es die Hydroxiphenylalkanolamin-Komponente in einer Retard- oder Depot-Form enthält.

**4.** Pharmazeutisches Kombinationspräparat nach den Ansprüchen 1 und 3, **dadurch gekennzeichnet**, daß es als Tablette, Dragée, Kapsel, Lösung oder Sirup ausgebildet ist.

**5.** Pharmazeutisches Kombinationspräparat nach den Ansprüchen 1 und 4, **dadurch gekennzeichnet**, daß es die Hydroxiphenylalkanolamin-Komponente in einer Menge von 2,5 bis 30 mg enthält.

## Claims

**1.** A pharmaceutical combination preparation, characterized in that it contains a mixture of one or more hydroxyphenylalkanolamines of the general formula:

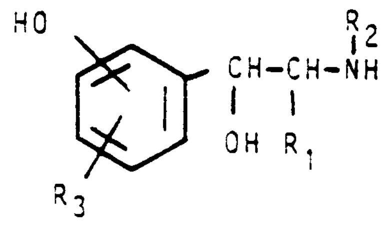

in which the OH group is In the m- or p-position and $R_1$ and $R_2$ are the same or different and represent hydrogen or a methyl, ethyl or isopropyl group and $R_3$ represents hydrogen or an hydroxyl, methyl or methoxy group; and vitamins $B_1$ and a magnesium salt.

**2.** A pharmaceutical combination preparation according to Claim 1, characterized in that it contains the hydroxyphenylalkanolamines as water-soluble salts.

**3.** A pharmaceutical combination preparation according to Claims 1 and 2, characterized in that it contains the hydroxyphenylalkanolamine component in a retard or sustained-release form.

**4.** A pharmaceutical combination preparation according to Claims 1 and 3, characterized in that it is in the form of a tablet, dragee, capsule, solution or syrup.

**5.** A pharmaceutical combination preparation according to Claims 1 and 4, characterized in that it contains the hydroxyphenylalkanolamine component in a quantity of from 2•5 to 30 mg.

**Revendications**

**1.** Préparation pharmaceutique sous forme de mélange, caractérisée en ce qu'elle contient un mélange d'une ou de plusieurs hydroxyphénylalcanolamines de formule générale

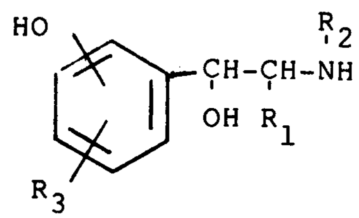

dans laquelle le groupe OH se trouve en position -m ou -p et $R_1$ et $R_2$ sont identiques ou différents et signifient l'hydrogène, un groupe méthyle, éthyle ou isopropyle et $R_3$ signifie l'hydrogène, un groupe hydroxyle, méthyle ou méthoxy, ainsi que de la vitamine $B_1$ et un sel de magnésium.

**2.** Préparation pharmaceutique sous forme de mélange selon la revendication 1, caractérisée en ce qu'elle contient les hydroxyphénylalcanolamines sous forme de sels hydrosolubles.

**3.** Préparation pharmaceutique sous forme de mélange selon les revendications 1 et 2, caractérisée en ce qu'elle contient le composant hydroxyphénylalcanolamine sous la forme retard ou à libération prolongée.

**4.** Préparation pharmaceutique sous forme de mélange selon les revendications 1 et 3, caractérisée en ce qu'elle se présente sous forme de comprimé, de dragée, de capsule, de solution ou de sirop.

**5.** Préparation pharmaceutique sous forme de mélange selon les revendications 1 et 4, caractérisée en ce qu'elle contient le composant hydroxyphénylalcanolamine en une quantité de 2,5 à 30 mg.